# FASCICULE DE BREVET EUROPEEN

(11) **EP 1 354 876 B1**
(45) Date de publication et mention de la délivrance du brevet: **27.04.2005**
(21) Numéro de dépôt: 03291420.2
(22) Date de dépôt: 13.06.2003
(51) Int. Cl.: C07D 209/42, C07K 5/02, C07K 5/06

(54) **Nouveau procédé de synthèse de l'acide (2S, 3aS, 7aS)-perhydroindole-2-carboxylique et de ses esters, et application à la synthèse du perindopril**
Verfahren zur Synthese von (2S,3aS,7aS)-Perhydroindol-2-carbonsäure und seiner Estern, und Verwendung in der Synthese von Perindopril
Method for synthesis of (2S,3aS,7aS)-perhydroindole-2-carboxylic acid and esters thereof; and use in the synthesis of perindopril

(43) Date de publication de la demande: 22.10.2003
(73) Titulaire: Les Laboratoires Servier, 92415 Courbevoie Cedex (FR)
(72) Inventeur: Dubuffet, Thierry, 76210 Bolbec (FR); Langlois, Pascal, 76210 Saint Jean de la Neuville (FR)

(56) Documents cités:
- EP-A- 0 308 339
- PYNE S. ET AL: "Asymmetric Synthesis of Proline Derivatives from (2R) and (2S)-2-tert-Butyl-3-Benzoyl-4-Methyleneoxa zolidin-5-one" TETRAHEDRON, ELSEVIER SCIENCE PUBLISHERS, AMSTERDAM, NL, vol. 51, no. 17, 1995, pages 5157-5168, XP002252296 ISSN: 0040-4020
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020

## Description

La présente invention concerne un procédé de synthèse de l'acide (2S, 3aS, 7aS)-perhydroindole-2-carboxylique et de ses esters, et leur application à la synthèse industrielle du perindopril et de ses sels pharmaceutiquement acceptables.

Plus spécifiquement, la présente invention concerne un nouveau procédé de synthèse des dérivés de formule (I) : 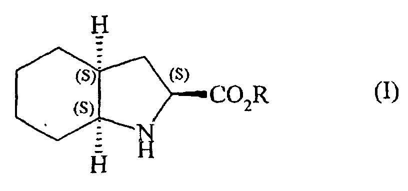 dans laquelle R représente un atome d'hydrogène ou un groupement alkyle (C₁-C₆) linéaire ou ramifié,
ainsi que leurs sels d'addition à un acide ou à une base minéral(e) ou organique.

Les composés de formule (1) obtenus selon le procédé de l'invention sont utiles dans la synthèse du perindopril de formule (II) : 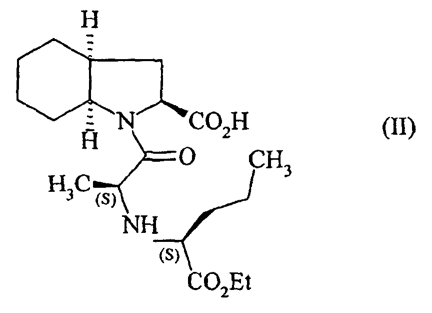 ainsi que dans celle de ses sels pharmaceutiquement acceptables.

Le perindopril, ainsi que ses sels, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse industrielle performant, permettant l'obtention sélective du diastéréoisomère (S,S,S) avec un bon rendement et une excellente pureté, à partir de matières premières bon marché.

Quelques méthodes de préparation des composés de formule (I) sont déjà connues.

Ainsi, le brevet EP 0 037 231 utilise comme matière première l'acide indole 2-carboxylique, qui est soumis à une hydrogénation catalytique sur rhodium pour donner un mélange des deux isomères cis endo de configurations respectives (2S, 3aS, 7aS) et (2R, 3aR, 7aR). Ce mélange est ensuite séparé de façon particulièrement laborieuse : synthèse du dérivé N-benzoylé, cristallisation fractionnée du sel du diastéréoisomère avec la (S)-α-phényl-éthylamine, libération des deux dérivés (S, S, S) et (R, R, R) N-benzoylés, puis élimination du groupement benzoyle, suivie d'un passage sur colonne échangeuse d'ions et d'une recristallisation.

Le brevet EP 0 115 345, pour cette même séparation, utilise plusieurs étapes nécessitant l'estérification de la fonction acide carboxylique par l'alcool benzylique, la salification de l'amino ester par la N-benzyloxycarbonyl-(S)-phénylalanine, la séparation par cristallisation fractionnée de l'isomère (S, S, S), la libération de la fonction aminée optionnellement suivie de la libération du groupement acide carboxylique.

Les brevets EP 0 308 339 et EP 0 308 341 utilisent également comme matière première l'acide indole 2-carboxylique, qui est dans un premier temps réduit en acide indoline 2-carboxylique, pour donner un mélange d'acide indoline carboxylique 2R et 2S, lesquels sont ensuite séparés par cristallisation fractionnée. L'isomère 2S est ensuite soumis à hydrogénation catalytique pour conduire au composé de formule (I).

La demanderesse a présentement mis au point un nouveau procédé de synthèse des dérivés de formule (I), à partir d'une matière première particulièrement bon marché, et qui permet l'obtention sélective du diastéréoisomère (S, S, S) avec un bon rendement et une excellente pureté.

Plus spécifiquement, la présente invention concerne un procédé de synthèse des composés de formule (I), caractérisé en ce que l'on condense la 2-(hydroxyméthyl)-cyclohexanone de formule (III) ; 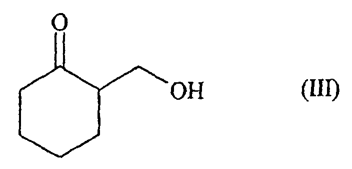 avec un ester de glycine de formule (IV) : 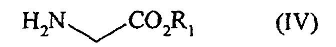 dans laquelle R₁ représente un groupement benzyle ou alkyle (C₁-C₆) linéaire ou ramifié,
en présence d'une base organique,
pour conduire après purification éventuelle au composé de formule (V), sous la forme d'un mélange racémique des composés de configurations (2S, 3aS) et (2R, 3aR) : 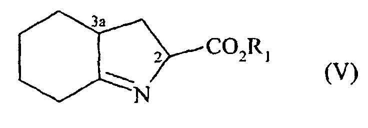 dans laquelle R₁ est tel que défini précédemment,
que l'on soumet à une réaction d'hydrogénation catalytique, pour conduire au composé de formule (VI), sous la forme d'un mélange racémique des configurations (2S, 3aS, 7aS) et (2R, 3aR, 7aR) : 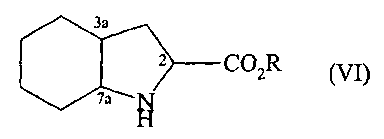 dans laquelle R est tel que défini dans la formule(I),
dont on isole l'isomère (2S, 3aS, 7aS) par résolution à l'aide d'une amine chirale, pour conduire au composé de formule (I).

Parmi les bases organiques utilisables dans le procédé selon l'invention, on peut citer à titre non limitatif les amines non cycliques telles que la triéthylamine, la diisopropylamine, la diisopropyléthylamine, et les amines cycliques telles que la pipéridine, la morpholine, la pyrrolidine, la pyridine.

La base organique préférée est la pipéridine.

Parmi les amines chirales utilisables dans le procédé selon l'invention, on peut citer à titre non limitatif la α-méthylbenzylamine, la 1-(1-naphtyl)-éthylamine, l'éphédrine, la α-chymotrypsine, la sec-butylamine, le 1-amino-2-méthylbutane, la N,N-diméthyl-1-phényléthylamine, la 1-cyclohexyléthylamine, la cyclosérine, la 2-(méthoxyméthyl)-pyrrolidine, le α-diméthylamino-ε-caprolactame, l'isobomylamine, la 1-(4-nitrophényl)-éthylamine, le α-amino-ε-caprolactame, le 2-amino-1-butanol, le 1-amino-2-propanol, la cinchonidine, la cinchonine, la N-méthyl-éphédrine, le phénylalaninol, la quinidine, le valinol, le α-phényl-glycinol, le leucinol.

Les amines chirales préférées sont la α-méthylbenzylamine et la 1-(1-naphtyl)-éthylamine.

Le composé de formule (I) ainsi obtenu a une très bonne pureté chimique et énantiomérique, ce qui rend son emploi particulièrement avantageux dans la synthèse du perindopril de formule (II).

A titre d'illustration, le couplage du composé de formule (I) obtenu selon le procédé de l'invention avec le composé de formule (VII) : 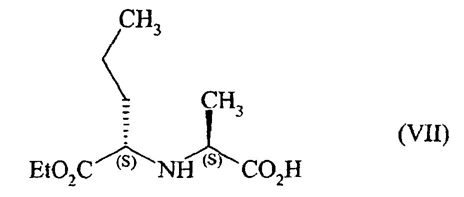 permet d'obtenir le perindopril de formule (II) avec une pureté et un rendement très satisfaisants.

### Définitions :

Par composé de configuration (2RS, 3aRS), on entend mélange racémique des composés de configurations absolues (2R, 3aR) et (2S, 3aS).
Par composé de configuration (2RS, 3aRS, 7aRS), on entend mélange racémique des composés de configurations (2R, 3aR, 7aR) et (2S, 3aS, 7aS).

### EXEMPLE : Acide (2S,3aS,7aS)-perhydroindole-2-carboxylique

### Stade A : Ester benzylique de l'acide (2RS, 3aRS,)-3,3a,4,5,6,7-hexahydro-2H-indole-2-carboxylique :

Dans un réacteur, charger 200 g de 2-(hydroxyméthyl)-cyclohexanone, 257 g de l'ester benzylique de la glycine, 200 ml d'éthanol et 6 ml de pipéridine. Après 2h d'agitation à température ambiante, porter le mélange réactionnel au reflux pendant 3h. Après retour à température ambiante, le précipité obtenu est filtré et purifié pour conduire au produit attendu.

### Stade B : Acide (2RS,3aRS, 7aRS)-perhydroindole-2-carboxylique :

Dans un hydrogénateur, placer 200 g du composé obtenu dans le stade précédent, en solution dans l'acide acétique, puis 5 g de Pd/C à 10 %. Hydrogéner sous pression de 4 bars à température ambiante, jusqu'à absorption de la quantité théorique d'hydrogène. Eliminer le catalyseur par filtration, puis évaporer le solvant. Le résidu ainsi obtenu est cristallisé, pour conduire au produit attendu.

### Stade C : Acide (2S,3aS, 7aS)-perhydroindole-2-carboxylique :

Dans un réacteur, charger 200 g du composé obtenu au stade précédent et 143 g de (R)-α-méthylbenzylamine, en solution dans l'éthanol.
On obtient un précipité blanc, que l'on filtre, puis recristallise.
Les cristaux obtenus sont dissous dans l'eau, puis de l'acide chlorhydrique en solution aqueuse est ajouté jusqu'à neutralisation.
Il se forme un précipité, qui est essoré, lavé puis séché, pour conduire au produit attendu avec une pureté chimique de 98% et une pureté énantiomérique de 99%.

## Revendications

1. Procédé de synthèse des composés de formule (I) : 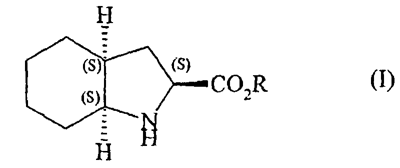 dans laquelle R représente un atome d'hydrogène ou un groupement alkyle (C₁-C₆) linéaire ou ramifié,
ainsi que leurs sels d'addition à un acide ou à une base minéral(e) ou organique,
**caractérisé en ce que** l'on condense la 2-(hydroxyméthyl)-cyclohexanone de formule (III) : 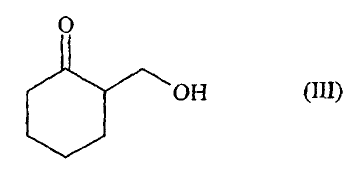 avec un ester de glycine de formule (IV) : 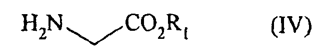 dans laquelle R₁ représente un groupement benzyle ou alkyle (C₁-C₆) linéaire ou ramifié,
en présence d'une base organique,
pour conduire après purification éventuelle au composé de formule (V), sous la forme d'un mélange racémique des composés de configurations (2S, 3aS) et (2R, 3aR) : 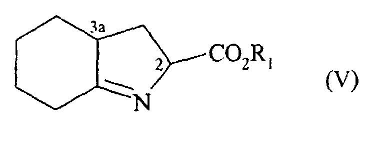 dans laquelle R₁ est tel que défini précédemment,
que l'on soumet à une réaction d'hydrogénation catalytique, pour conduire au composé de formule (VI), sous la forme d'un mélange racémique des configurations (2S, 3aS, 7aS) et (2R, 3aR, 7aR) : 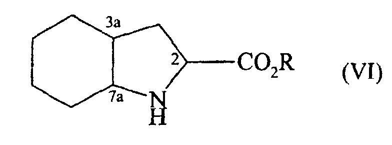 dans laquelle R est tel que défini dans la formule(I),
dont on isole l'isomère (2S, 3aS, 7aS) par résolution à l'aide d'une amine chirale, pour conduire au composé de formule (I).

2. Procédé de synthèse selon la revendication 1, **caractérisé en ce que** l'amine chirale est la α-méthylbenzylamine ou la 1-(1-naphtyl)-éthylamine.

3. Procédé de synthèse du perindopril ou de ses sels pharmaceutiquement acceptables à partir du composé de formule (I), **caractérisé en ce que** ledit composé de formule (I) est obtenu selon le procédé de l'une quelconque des revendications 1 ou 2.

## Patentansprüche

1. Verfahren zur Synthese von Verbindungen der Formel (I): 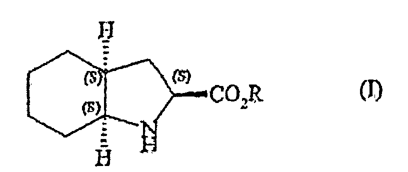 in der R ein Wasserstoffatom oder eine geradkettige oder verzweigte (C₁-C₆)-Alkylgruppe bedeutet,
sowie von deren Additionssalzen mit einer anorganischen oder organischen Säure oder Base,
**dadurch gekennzeichnet. daß** man 2-(Hydroxymethyl)-cyclohexanon der Formel (III): 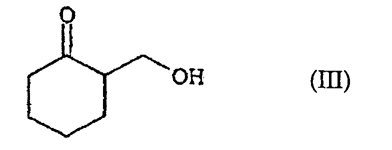 mit einem Glycinester der Formel (IV): 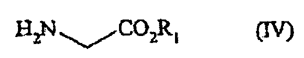 in der R₁ eine Benzylgruppe oder eine geradkettige oder verzweigte (C₁-C₆)-Alkylgruppe bedeutet,
in Gegenwart einer organischen Base kondensiert, so daß man nach der eventuellen Reinigung die Verbindung der Formel (V) in Form einer racemischen Mischung der Verbindungen der Konfigurationen (2S, 3aS) und (2R, 3aR) erhält: 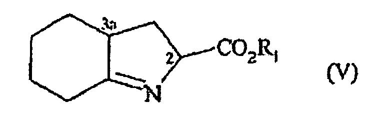 in der R₁ die oben angegebenen Bedeutungen besitzt, welche man einer katalytischen Hydrierungsreaktion unterwirft, so daß man die Verbindung der Formel (VI) in Form einer racemischen Mischung der Verbindungen der Konfigurationen (2S, 3aS, 7aS) und (2R, 3aR, 7aR) erhält: 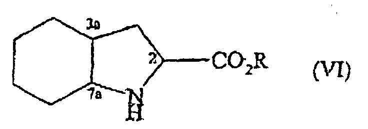 in der R die bezüglich der Formel (I) angegebenen Bedeutungen besitzt,
aus der man durch Aufspaltung mit Hilfe eines chiralen Amins das (2S, 3aS, 7aS)-Isomere isoliert unter Erhalt der Verbindung der Formel (I).

2. Syntheseverfahren nach Anspruch 1, **dadurch gekennzeichnet, daß** das chirale Amin α-Methylbenzylamin oder 1-(1-Naphthyl)-ethylamin ist.

3. Verfahren zur Synthese von Perindopril oder von seinen pharmazeutisch annehmbaren Salzen ausgehend von der Verbindung der Formel (I), **dadurch gekennzeichnet, daß** die Verbindung der Formel (I) nach dem Verfahren nach einem der Ansprüche 1 oder 2 hergestellt wird.

## Claims

1. Process for the synthesis of compounds of formula (1) : 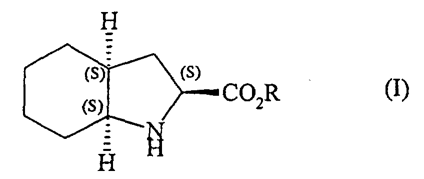 wherein R represents a hydrogen atom or a linear or branched (C₁-C₆)alkyl group,
and addition salts thereof with a mineral or organic acid or base,
**characterised in that** 2-(hydroxymethyl)-cyclohexanone of formula (III) : 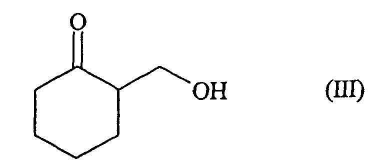 is condensed with a glycine ester of formula (IV) : 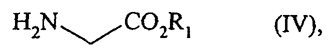 wherein R₁ represents a benzyl or linear or branched (C₁-C₆)alkyl group,
in the presence of an organic base
to yield, after optional purification, a compound of formula (V) in the form of a racemic mixture of the compounds having the (2S,3aS) and (2R,3aR) configurations: 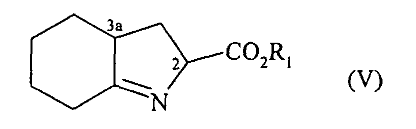 wherein R₁ is as defined hereinbefore,
which is subjected to a catalytic hydrogenation reaction to yield a compound of formula (VI) in the form of a racemic mixture of the (2S,3aS,7aS) and (2R,3aR,7aR) configurations: 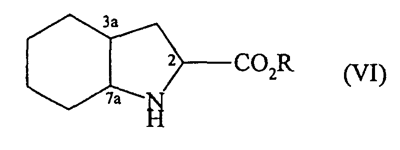 wherein R is as defined for formula (I),
the (2S,3aS,7aS) isomer of which is isolated, by resolution using a chiral amine, to yield a compound of formula (I).

2. Synthesis process according to claim 1, **characterised in that** the chiral amine is α-methylbenzylamine or 1-(1-naphthyl)-ethylamine.

3. Process for the synthesis of perindopril or pharmaceutically acceptable salts thereof starting from a compound of formula (I), **characterised in that** the said compound of formula (I) is obtained according to the process of either claim 1 or claim 2.
